# EUROPÄISCHE PATENTSCHRIFT

(11) **EP 1 579 812 B1**
(45) Veröffentlichungstag und Bekanntmachung des Hinweises auf die Patenterteilung: **09.07.2008**
(21) Anmeldenummer: 04007070.8
(22) Anmeldetag: 24.03.2004
(51) Int. Cl.: A61B 17/15

(54) **Vorrichtung zum Setzen eines Schnittblocks für eine Resektion der Tibia**
Device for positioning a cutting guide for tibial resection
Dispositif pour la mise en place d'un guide de coupe pour la résection du tibia

(43) Veröffentlichungstag der Anmeldung: 28.09.2005
(73) Patentinhaber: Zimmer GmbH, 8404 Winterthur (CH); Omnex, 14000 Caen (FR)
(72) Erfinder: Houdemer, Hervé, 8400 Winterthur (CH); Leclercq, Sylvian, 14990 Bernieres sur mer (FR); Coudane, Henry, 5400 Nancy (FR); Augereau-Vacher, Bernard, 9220 Neuilly (FR); Huten, Denis, 94300 Vincenne (FR); Nizard, Rémy, 75011 Paris (FR); Beaufils, M., 78000 Versailles (FR)
(74) Vertreter: Manitz, Finsterwald & Partner GbR

(56) Entgegenhaltungen:
- FR-A- 2 703 584
- FR-A- 2 770 765
- FR-A- 2 776 176
- US-A- 4 952 213
- US-A- 5 342 368
- US-A- 5 681 316

## Beschreibung

Die Erfindung betrifft eine Vorrichtung zum Setzen eines Schnittblocks für eine Resektion der Tibia, welche sowohl extramedullär als auch intramedullär an der Tibia verankerbar ist und ein insbesondere rohrförmiges Verstellorgan aufweist, mit welchem ein zur Verankerung an einer natürlichen Tibiaplattform vorgesehener Querarm verbunden ist, wobei der Schnittblock an einer ersten Geradführung relativ zum Querarm in seiner Höhe verstellbar ist.

Bei der Resektion von natürlichen Tibiaplattformen werden Schnittblöcke, die zur Führung von Sägeblättern dienen, mittels Stiften an der Tibia befestigt. Die Schnittblöcke werden mittels einer an der Tibia verankerbaren, grundsätzlich bekannten Vorrichtung der eingangs genannten Art ausgerichtet, wobei die Verankerung der Vorrichtung entweder extramedullär außerhalb des Markraums oder intramedullär mit Hilfe eines in Richtung der Tibiaachse orientierten Marknagels erfolgt.

Bei der extramedullären Verankerung ist das Verstellorgan z.B. als Verstellhülse ausgebildet, in die ein Verlängerungsstab eingeführt ist. Die bekannte Vorrichtung stützt sich dabei mit dem Querarm an einem Punkt nahe der Eminentia der natürlichen Tibiaplattform und mit dem in die Verstellhülse eingeführten Stab über einen vereinfachten Kreuzschlitten an der Tibia im Bereich des Knöchels ab. Mit einer Verschiebung des Kreuzschlittens entlang der transversalen Achse kann eine Korrektur einer Varus-/Valgusstellung und mit einer Verschiebung entlang der sagittalen Achse eine gegenüber der Transversalebene veränderte Neigung des Schnittblocks bzw. des Querarms erreicht werden. Ist die Vorrichtung korrekt ausgerichtet, werden der Querarm und damit die gesamte Vorrichtung mittels eines Stifts an der Tibia fixiert.

Bei der intramedullären Verankerung mit einem durch eine Führungsbohrung des Querarms entlang der Tibiaachse in die Tibia eingeschlagenen Marknagel kann die bekannte Vorrichtung zum Setzen eines Schnittblocks um die Längsachse des Marknagels gedreht werden. In Analogie zur extramedullären Verankerung wird nach korrekter Ausrichtung der Vorrichtung der Querarm mittels eines Stifts fixiert.

Der Schnittblock ist an der ersten Geradführung der Vorrichtung parallel zur Längsachse der Verstellhülse geführt und wird durch eine Verstellmutter in seiner jeweiligen Höhe abgestützt. Die für den Resektionsschnitt korrekte Höhe des Schnittblocks wird mit einem auf die Vorrichtung aufsetzbaren Tiefentaster bestimmt, der die tiefsten Punkte der Tibiakondylen antastet. Anschließend wird der Schnittblock über darin ausgebildete Führungsbohrungen mittels zweier Verankerungsstifte entlang der sagittalen Achse verschiebbar an der Tibia fixiert.

Die Neigung des Schnittblocks gegenüber der transversalen Achse und die Neigung gegen posterior kann mit einer nachträglich am Schnittblock befestigbaren Visierstange kontrolliert werden. Eine ggf. erforderliche Korrektur der Neigung des Schnittblocks gegenüber der transversalen Achse um bis zu 2° ist mittels des Schnittblocks möglich, wenn einer der Verankerungsstifte in eine gegenüber der ursprünglich vorgesehenen Führungsbohrung verschwenkte Führungsbohrung des Schnittblocks eingesetzt wird.

Mittels einer im Schnittblock ausgebildeten Schrägbohrung, die gegen die sagittale Achse geneigt ist, und eines in der Schrägbohrung geführten dritten Stifts, der in die Tibia eingeschlagen wird, kann schließlich auch die Position des Schnittblocks entlang der sagittalen Achse fixiert werden.

Danach kann auf den korrekt ausgerichteten und an der Tibia befestigten Schnittblock eine Sägeblattführung aufgesetzt und mittels eines Sägeblatts die Resektion der natürlichen Tibiaplattform durchgeführt werden.

Nachteilig bei derartigen Vorrichtungen ist jedoch, dass bei intramedullärer Verankerung die Vorrichtung lediglich um die Längsachse des Marknagels gedreht und bei extramedullärer Verankerung die Ausrichtung der Vorrichtung nur mittels eines zusätzlichen Kreuzschlittens ausgeführt werden kann.

US 5,681,316 beschreibt eine Vorrichtung zum Setzen eines Schnittblocks, welche intramedullär an der Tibia verankerbar ist. Hierzu weist die Vorrichtung eine Hülse auf, mit welcher ein zur Verankerung an einer natürlichen Tibiaplattform vorgesehener Querarm verbunden ist. Der Schnittblock ist an einer ersten Geradführung relativ zum Querarm in seiner Höhe verstellbar. Für die erste Geradführung ist eine Gelenkanordnung vorgesehen, durch die unterschiedliche Relativstellungen zwischen der ersten Geradführung und dem Querarm bzw. der Hülse realisierbar sind. Die Gelenkanordnung umfasst eine in anterior-/posterior-Richtung orientierte Schwenkachse und eine in medio-lateraler Richtung orientierte Schwenkachse. Ein horizontales Befestigungsteil, mit welchem die erste Geradführung verbunden ist, ist um die in medio-lateraler Richtung orientierte Schwenkachse relativ zum Querarm verschwenkbar.

Der Erfindung liegt die Aufgabe zugrunde, eine Vorrichtung der eingangs genannten Art zu schaffen, die ein einfaches und zugleich präzises Ausrichten des Schnittblocks an der zu resezierenden Tibia und insbesondere die Korrektur einer Fehlstellung der Beine ermöglicht.

Die Lösung dieser Aufgabe erfolgt durch die Merkmale des Anspruchs 1.

Bei der erfindungsgemäßen Vorrichtung zum Setzen eines Schnittblocks ist vorgesehen, dass von den Elementen Geradführung, Querarm und Verstellorgan zumindest ein Element relativ zu den beiden anderen Elementen verstellbar ist. Hierdurch wird ermöglicht, dass die Geradführung und damit auch ein an der Geradführung geführter Schnittblock relativ zu der Tibia verstellt werden kann.

Die erfindungsgemäße Vorrichtung besitzt insbesondere den Vorteil, dass durch die Gelenkanordnung auch bei intramedullärer Verankerung eine Korrektur einer Varus-/Valgusstellung und/oder eine gegenüber der sagittalen Achse veränderte Neigung des Schnittblocks erreicht werden kann. Bei extramedullärer Verankerung der Vorrichtung kann auf einen entlang der transversalen und entlang der sagittalen Achse verschiebbaren Kreuzschlitten verzichtet werden.

Vorteilhafte Ausführungsformen der Erfindung sind in den Unteransprüchen, der Beschreibung sowie der Zeichnung angegeben.

Die Gelenkanordnung umfasst wenigstens zwei Schwenkachsen, die insbesondere senkrecht zueinander orientiert sein können. Die erste Schwenkachse ist in anterior-/posterior-Richtung und die zweite Schwenkachse in medio-lateraler Richtung orientiert, so dass durch die erste Schwenkachse eine Korrektur einer Varus-/Valgusstellung und durch die zweite Schwenkachse eine gegenüber der sagittalen Achse veränderte Neigung des Schnittblocks erreicht werden kann.

Ein besonders kompakter Aufbau der Gelenkanordnung ergibt sich, wenn gemäß einem Ausführungsbeispiel der Erfindung die Schwenkachsen oberhalb der Geradführung verlaufen. Des Weiteren können sich die Schwenkachsen schneiden, so dass durch den Schnittpunkt der Schwenkachsen ein zentraler Drehpunkt der Gelenkanordnung vorhanden ist.

Der Querarm umfasst einen Querträger und ein um eine erste Achse relativ zum Querträger schwenkbares erstes Schwenkelement, das an der natürlichen Tibiaplattform verankerbar ist, wobei relativ zum Querarm ein zweites Schwenkelement um eine zweite Achse verschwenkbar ist, mit welchem die erste Geradführung verbunden ist.

In einer weiteren bevorzugten Ausführungsform sind der Querträger und das Verstellorgan miteinander verbunden, so dass bei einer Schwenkbewegung des Querträgers um die erste Achse auch das Verstellorgan relativ zu dem Schwenkelement verschwenkt wird. Das Verstellorgan kann als Visierstange zur Kontrolle der Neigung des Schnittblocks gegenüber der transversalen Achse verwendet werden.

Besonders vorteilhaft ist es, wenn zwischen der ersten Geradführung zur Höhenverstellung des Schnittblocks und dem zweiten Schwenkelement eine zweite Geradführung zur Verstellung des Schnittblocks in anterior/ posterior-Richtung angebracht ist. Hierdurch kann der Schnittblock nach der Ausrichtung der Vorrichtung zum Setzen des Schnittblocks direkt an die Tibia angelegt werden, so dass die Befestigung des Schnittblocks an der Tibia erleichtert ist.

Ferner kann erfindungsgemäß vorgesehen sein, dass der Winkel einer Schwenkbewegung um eine erste und/oder zweite Achse auf einer Skala ablesbar ist, so dass durch einen Vergleich der bei der Operation abgelesenen Winkel mit bei der Operationsplanung mittels Röntgenbildern bestimmten Winkeln die korrekte Ausrichtung der Vorrichtung überprüft werden kann.

Der Winkel einer Schwenkbewegung um eine erste und/oder zweite Achse ist bevorzugt mit Formschluss oder Kraftschluss festsetzbar. Dies ermöglicht, dass bei korrekt ausgerichteter Vorrichtung der Querträger, das erste Schwenkelement und das zweite Schwenkelement in ihrer jeweiligen Stellung arretiert werden können, d.h. die Gelenkanordnung bei der gewünschten Relativstellung ihrer Bestandteile festgesetzt werden kann.

Das erste Schwenkelement weist bevorzugt zwei im gesetzten Zustand gegen distal vorstehende Stifte zur Verankerung in der natürlichen Tibiaplattform und eine Führungsbohrung für den Fall einer Verankerung mit Ausrichtung an einem intramedullären Nagel auf. Die korrekt ausgerichtete Setzvorrichtung kann bei extramedullärer Verankerung durch die beiden Stifte und bei intramedullärer Verankerung durch einen der beiden Stifte und den intramedullären Nagel fixiert werden.

Das erste Schwenkelement ist zumindest zum Teil in Form eines Rahmens ausgeführt sein, welcher den Querträger einschließt. Der Querträger ist somit gewissermaßen als ein Kern ausgebildet, der von dem rahmenförmigen Schwenkelement zumindest bereichsweise umgeben ist, wodurch ein besonders Platz sparender Aufbau des Querarms gewährleistet ist.

Um zu ermöglichen, dass der Querträger und das erste Schwenkelement in einer bestimmten Relativstellung zueinander arretiert werden können, kann der Querträger des Querarms mit einem in Achsrichtung des Verstellorgans vorstehenden ersten Winkelstück versehen sein, das mit einem vom ersten Schwenkelement vorstehenden zweiten Winkelstück verriegelbar ist.

Die Schwenkbewegung des zweiten Schwenkelements relativ zum Querarm kann besonders einfach dadurch realisiert werden, dass das zweite Schwenkelement ein in einem Schlitz des Querträgers geführtes und schwenkbares Flachstück umfasst, welches in eine Basisplatte übergeht.

Des Weiteren wird erfindungsgemäß vorgeschlagen, dass an der Basisplatte eine Schlittenführung in anterior-/posterior-Richtung für ein als Schlitten ausgeführtes Trägerelement der ersten Geradführung für den Schnittblock vorhanden ist. Durch die Schlittenführung kann ein in der ersten Geradführung geführter Schnittblock auf die Tibia zu oder von dieser weg bewegt werden.

Es ist weiterhin bevorzugt, dass die beiden in medio-lateraler Richtung gelegenen Enden des Schnittblocks voneinander verschieden ausgebildet sind. Insbesondere kann das eine Ende einen gegen posterior vorstehenden Wulst aufweisen. Hierdurch kann einerseits die Auflagefläche für ein Sägeblatt vergrößert und andererseits eine Anpassung an die anatomische Form der Tibia erreicht werden.

In einer besonders bevorzugten Ausführungsform sind obere und untere Auflageflächen des Schnittblocks sowie im Schnittblock ausgebildete Führungsbohrungen für Verankerungsstifte bezüglich einer transversalen Mittelebene des Schnittblocks symmetrisch ausgebildet. Hierdurch kann durch Wenden des Schnittblocks der Schnittblock und die Setzvorrichtung auf einfache Weise sowohl bei einer linken als auch einer rechten Tibia eingesetzt werden.

Es ist weiterhin bevorzugt, dass der Schnittblock Führungsbohrungen für eine Sägeblattführung aufweist. Dies ermöglicht, dass auf den Schnittblock eine Sägeblattführung aufgesetzt werden kann, die eine exakte Führung des Sägeblatts bei der Resektion der Tibia gewährleisten kann.

Die Erfindung wird im Folgenden beispielhaft unter Bezugnahme auf die Zeichnung beschrieben. Es zeigen:
- Fig. 1: eine perspektivische Ansicht einer erfindungsgemäßen Setzvorrichtung mit einem Schnittblock,
- Fig. 2: eine Seitenansicht der Vorrichtung von Fig. 1,
- Fig. 3: eine Draufsicht der Vorrichtung von Fig. 1,
- Fig. 4: eine gemäß Fig. 3 längsgeschnittene Seitenansicht der Vorrichtung von Fig. 1,
- Fig. 5: ein erstes Winkelstück der erfindungsgemäßen Setzvorrichtung von posterior betrachtet,
- Fig. 6: eine vergrößerte Darstellung des Details A aus Fig. 4,
- Fig. 7: eine perspektivische Ansicht eines schematisch dargestellten erfindungsgemäßen Schnittblocks,
- Fig. 8 a, b: verschiedene Ansichten einer Sägeblattführung, und
- Fig. 9: einen Querschnitt durch einen erfindungsgemäßen Schnittblock mit aufgesetzter Sägeblattführung.

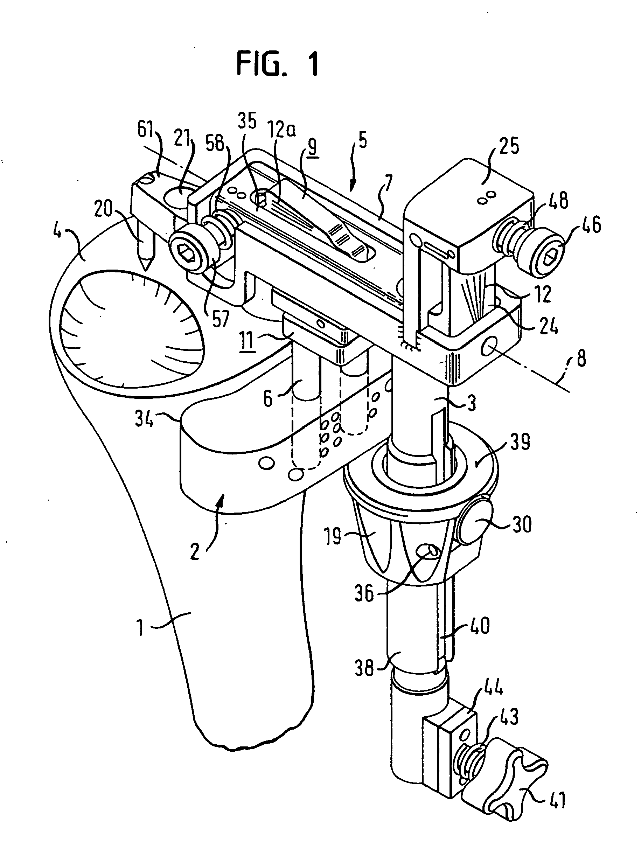

Fig. 1 zeigt eine an einer natürlichen Tibiaplattform 4 zu verankernde erfindungsgemäße Vorrichtung zum Setzen eines Schnittblocks 2 für eine Resektion der Tibia 1. Gemäß den Fig. 1 bis 4 umfasst die erfindungsgemäße Vorrichtung eine Verstellhülse 3, einen mit der Verstellhülse 3 verbundenen Querarm 5 und eine erste Geradführung 6, die zwei Stäbe umfasst und an der der Schnittblock 2 geführt ist.

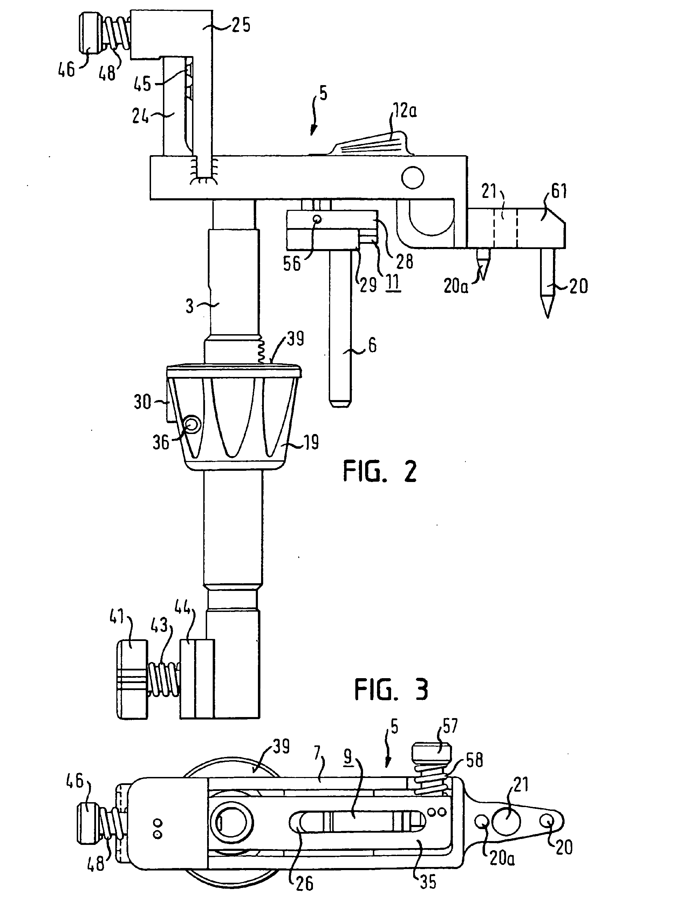

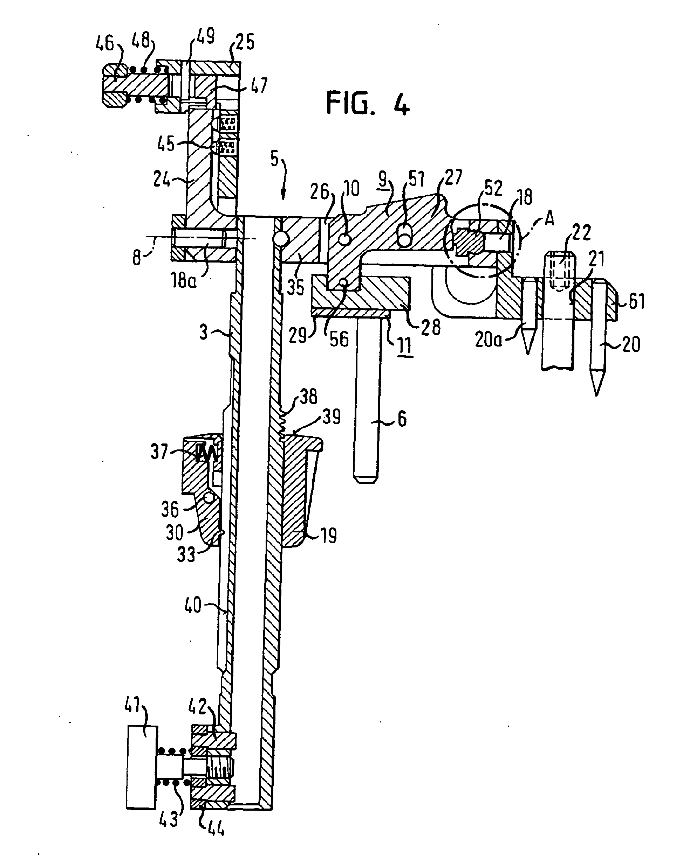

Der Schnittblock 2 ist relativ zum Querarm 5 in seiner Höhe verstellbar und wird durch eine Verstellmutter 19, die an der Verstellhülse 3 befestigt und entlang dieser verstellbar ist, über eine an der Verstellmutter 19 ausgebildete Stützfläche 39 in der jeweiligen Höhe gehalten. Die Verstellmutter 19 kann dabei mittels eines an der Verstellhülse 3 ausgebildeten Gewindes 38, das in den Fig. 2 und 4 der Einfachheit halber nur ausschnittsweise dargestellt ist, durch eine Schraubdrehung oder durch Betätigung eines Kipphebels 30 verstellt werden.

Gemäß Fig. 4 weist die Verstellmutter 19 kein durchgehendes Innengewinde, sondern lediglich einen an dem Kipphebel 30 angebrachten Zahn 33 auf, der bei nicht betätigtem Kipphebel 30 in das Gewinde 38 eingreift, so dass eine Schraubdrehung der Verstellmutter 19 ermöglicht ist. Bei Betätigung des Kipphebels 30 wird der Eingriff des Zahns 33 der Verstellmutter 19 in das Gewinde 38 der Verstellhülse 3 gegen die Rückstellkraft einer Rückstellfeder 37 gelöst, so dass die Verstellmutter 19 entlang der Verstellhülse 3 verschoben werden kann. Der Kipphebel 30 führt dabei eine Schwenkbewegung um eine Achse 36 aus.

Das Verstellen der Verstellmutter 19 durch Schraubdrehung stellt eine stufenlose Feinjustierung, durch Betätigung des Kipphebels 30 eine gerasterte Grobjustierung dar. In einer in der Verstellhülse 3 ausgebildeten Nut 40 kann eine Skala zum Ablesen der Höhe, in der sich die Verstellmutter 19 jeweils befindet, angebracht sein.

Der Querarm 5 umfasst gemäß Fig. 1 ein erstes Schwenkelement 7 und einen Querträger 35, der fest mit der Verstellhülse 3 verbunden ist. Das erste Schwenkelement 7, das zumindest bereichsweise rahmenförmig ausgeführt ist und den insofern einen Kern bildenden Querträger 35 umschließt, ist um eine erste Achse 8, die in anterior-posterior Richtung orientiert ist, relativ zu dem Querträger 35 und relativ zu der Verstellhülse 3 verschwenkbar. Das erste Schwenkelement 7 ermöglicht somit eine Korrektur der Neigung der ersten Geradführung 6 und damit des Schnittblocks 2 gegenüber der transversalen Achse. Die erste Achse 8 ist an den beiden in anterior-/posterior gelegenen Enden des Querarms 5 jeweils durch einen Stift 18, 18a realisiert (Fig. 4).

Das erste Schwenkelement 7 weist eine stufenartig nach unten versetzte, nasenartige Verlängerung 61 auf, welche zur Verankerung der erfindungsgemäßen Vorrichtung an der natürlichen Tibiaplattform 4 dient. Bei extramedullärer Verankerung sind zwei Stifte 20, 20a vorgesehen, die im gesetzten Zustand, der jeweils in den Fig. 1, 2 und 4 dargestellt ist, gegen distal vorstehen. Wie insbesondere in Fig. 4 gezeigt ist, kann bei intramedullärer Verankerung einer der beiden Stifte 20, 20a durch einen intramedullären Nagel 22 ersetzt werden, der sich durch eine in der Verlängerung 61 ausgebildete Führungsbohrung 21 erstreckt.

An dem Querträger 35 ist ein in Achsrichtung der Verstellhülse 3 nach oben vorstehendes erstes Winkelstück 24 ausgebildet, das mit einem zweiten Winkelstück 25 verriegelbar ist, welches von dem ersten Schwenkelement 7 ebenfalls nach oben absteht. Der Verriegelungsmechanismus wird über einen Druckknopf 46 gesteuert, der in das zweite Winkelstück 25 eingesetzt ist und durch eine Rückstellfeder 48 in die in den Fig. 1 bis 4 dargestellte verriegelnde Position gedrückt wird, in der er durch Sicherungsstifte 49 gehalten wird (Fig. 4).

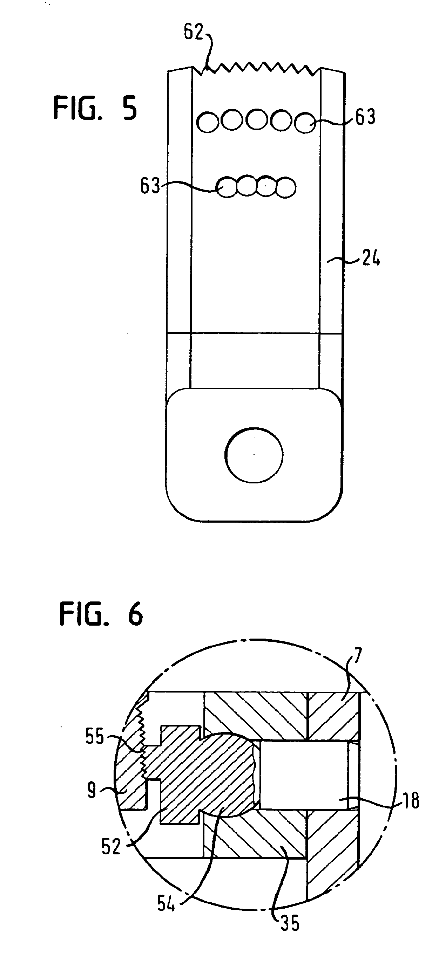

Die Verriegelung der beiden Winkelstücke 24, 25 erfolgt über eine an dem Druckknopf 46 ausgebildete Klinke 47, die eine nicht dargestellte Verzahnung aufweist, in die eine entsprechende Verzahnung 62 des ersten Winkelstück 24 eingreift (Fig. 5). Zum Lösen der Verriegelung wird der Druckknopf 46 betätigt, so dass die Klinke 47 verschoben und der Eingriff der Verzahnung der Klinke 47 in die Verzahnung 62 des ersten Winkelstücks 24 gelöst wird.

Nur in der nicht verriegelnden Position des Druckknopfs 46 ist das erste Schwenkelement 7 um die erste Achse 8 relativ zu dem Querträger 35 und relativ zu der Verstellhülse 3 verschwenkbar. Gemäß Fig. 5 sind zum Einstellen des Winkels der Schwenkbewegung um die erste Achse 8 in dem ersten Winkelstück 24 zwei übereinander liegende Reihen bogenförmig angeordneter sphärischer Vertiefungen 63 ausgebildet, in die jeweils mittels einer Feder vorgespannte, an dem zweiten Winkelstück 25 gehaltene Einrastkugeln 45 einrasten können (Fig. 2, 4). Der Winkel der Schwenkbewegung um die erste Achse 8 ist auf einer an dem ersten Winkelstück 24 angebrachten Skala 12 ablesbar (Fig. 1).

Die Setzvorrichtung für den Schnittblock 2 umfasst ferner ein zweites Schwenkelement 9, das um eine in medio-lateraler Richtung orientierte, zweite Achse 10 (Fig. 4) relativ zu dem Querträger 35 verschwenkbar ist. Das zweite Schwenkelement 9 ist mit der ersten Geradführung 6 verbunden, so dass durch eine Schwenkbewegung des zweiten Schwenkelements 9 um die zweite Achse 10 eine Korrektur der Neigung der ersten Geradführung 6 und damit des Schnittblocks 2 gegenüber der sagittalen Achse ermöglicht wird.

Das zweite Schwenkelement 9 ist als Flachstück 27 ausgebildet, das in einen den Querträger 35 in superior-/inferior-Richtung durchsetzenden Schlitz 26 des Querträgers 35 eingesetzt ist und in eine Basisplatte 28 übergeht, die mit einem Trägerelement 29 der ersten Geradführung 6 verbunden ist (Fig. 4). Das Flachstück 27 und die Basisplatte 28 sind durch einen Kerbstift 56 miteinander verbunden.

Zwischen der Basisplatte 28 des zweiten Schwenkelements 9 und dem als Schlitten ausgebildeten Trägerelement 29 der ersten Geradführung 6 ist eine zweite Geradführung 11 zur Verstellung des Schnittblocks 2 in anterior-/posterior-Richtung vorgesehen, wie insbesondere aus Fig. 2 ersichtlich ist. Mittels der Schlittenführung 11 kann der Schnittblock 2 direkt an die Tibia 1 "herangefahren" werden.

Das zweite Schwenkelement 9 und der Querträger 35 können miteinander verriegelt werden. Die Verriegelung wird durch einen Druckknopf 57 gesteuert, der in den Querträger 35 eingesetzt ist und durch eine Rückstellfeder 58 in die in den Fig. 1 und 3 dargestellte verriegelnde Position gedrückt wird. Gemäß Fig. 6 weist der Druckknopf 57 einen teilzylindrischen, im Querträger 35 verschiebbar gelagerten Schaft 54 auf, an dem eine Klinke 52 mit einer Verzahnung 55 angebracht ist. Die Verzahnung 55 der Klinke 52 greift in eine entsprechend ausgebildete Verzahnung des zweiten Schwenkelements 9 ein, so dass der Querträger 35 und das zweite Schwenkelement 9 effektiv miteinander verriegelt sind.

Zum Lösen der Verriegelung wird der Druckknopf 57 in Richtung des Querträgers 35 gedrückt, so dass die Klinke 52 in medio-lateraler Richtung verschoben und der Eingriff der Verzahnung 55 der Klinke 52 in die Verzahnung des zweiten Schwenkelements 9 gelöst wird. Der zweite Schwenkhebel 9 ist in dieser Stellung des Druckknopfs 57 um die zweite Achse 10 verschwenkbar, wobei die Schwenkbewegung durch das Zusammenwirken eines in dem zweiten Schwenkelement 9 ausgebildeten Langlochs 51 mit einem in das Langloch 51 eingesetzten Stift begrenzt ist (Fig. 4). Der Winkel der Schwenkbewegung um die zweite Achse 10 ist auf einer an dem zweiten Schwenkelement 9 angebrachten Skala 12a ablesbar.

Zur extramedullären Verankerung der Vorrichtung kann in die Verstellhülse 3 ein nicht dargestellter Verlängerungsstab eingeführt werden, der im Bereich des Knöchels an der Tibia 1 abgestützt wird. Zur Befestigung des Verlängerungsstabs ist eine Klemmschraube 41 vorgesehen. Beim Einschrauben der Klemmschraube 41 in die Verstellhülse 3 werden eine Platte 44 und an der Platte 44 angebrachte Anpressbacken 42 mittels der Federkraft einer Klemmfeder 43 in Richtung des in die Verstellhülse 3 eingeführten Verlängerungsstabs gedrückt, so dass dieser durch die Anpressbacken 42 fest gehalten wird (Fig. 4).

Die in anterior-/posterior-Richtung orientierte Achse 8 und die in medio-lateraler Richtung orientierte Achse 10 sind senkrecht zueinander orientiert, jeweils oberhalb der Geradführung 6 gelegen und derart angeordnet, dass sie sich schneiden. Durch die Achsen 8, 10, die eine Gelenkanordnung bilden, werden unterschiedliche, praktisch beliebig einstellbare Relativorientierungen zwischen der Geradführung 6, dem Querträger 5 bzw. der Verstellhülse 3 und dem ersten Schwenkelement 7 ermöglicht.

Die Gelenkanordnung der erfindungsgemäßen Setzvorrichtung ermöglicht damit eine beliebige Einstellbarkeit der Neigung des Schnittblocks 2 gegenüber einer Transversalebene der Tibia 1. Insbesondere kann der Schnittblock 2 gegenüber jeder in der Transversalebene verlaufenden Geraden geneigt werden. Durch die als eine Art kompaktes Gelenk realisierte Gelenkanordnung kann die Setzvorrichtung besonders einfach gehandhabt werden. Ansonsten kann die Setzvorrichtung analog zu einer aus dem Stand der Technik bekannten Vorrichtung, wie sie vorstehend erläutert ist, verwendet werden.

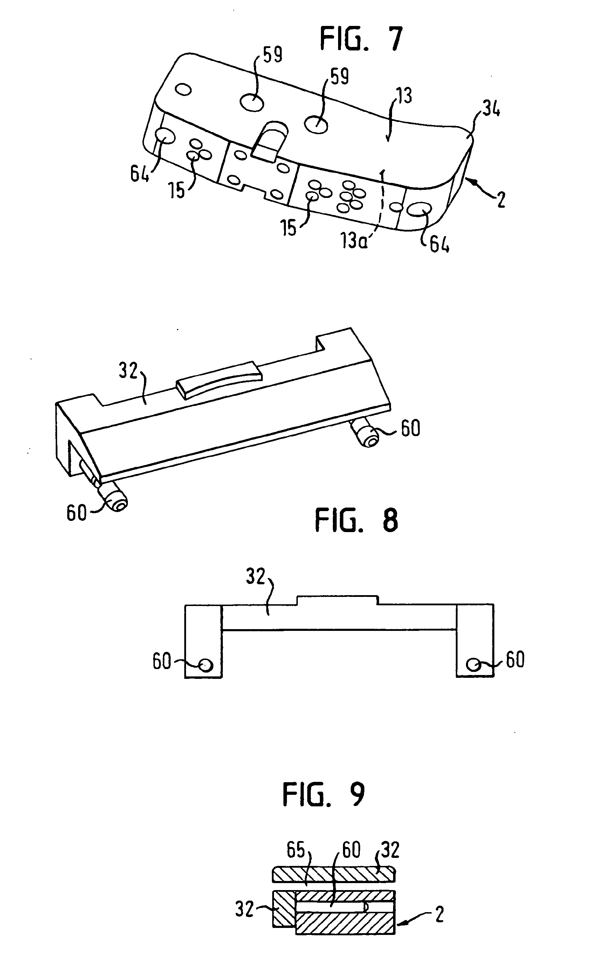

Zur Aufnahme der beiden Stäbe der ersten Geradführung 6 weist der in Fig. 7 dargestellte erfindungsgemäße Schnittblock 2 zwei den Schnittblock 2 in superior-/inferior-Richtung durchsetzende Führungsbohrungen 59 auf. In dem Schnittblock 2 sind weiterhin mehrere in anterior-/posterior-Richtung verlaufende Führungsbohrungen 15 ausgebildet, die eine Verankerung des Schnittblocks an der Tibia 1 mittels nicht dargestellter Verankerungsstifte ermöglichen.

Die Führungsbohrungen 15 zur Aufnahme der Verankerungsstifte sowie obere und untere Auflageflächen 13, 13a des Schnittblocks 2 sind bezüglich einer transversalen Mittelebene des Schnittblocks 2 symmetrisch ausgebildet, so dass der Schnittblock 2 sowohl bei einer Resektion einer linken als auch einer rechten Tibia 1 einsetzbar ist. Die beiden in medio-lateraler Richtung gelegenen Enden des Schnittblocks 2 hingegen sind voneinander verschieden ausgebildet, wobei das eine Ende einen gegen posterior vorstehenden Wulst 34 aufweist, so dass eine vergrößerte Auflagefläche 13, 13a gebildet ist.

Der Schnittblock 2 weist ferner zwei Führungsbohrungen 64 auf, die das Aufsetzen einer in den Fig. 8a und 8b dargestellten Sägeblattführung 32 ermöglichen. Hierfür umfasst die Sägeblattführung 32 zwei Zylinderstifte 60, die in die beiden Führungsbohrungen 64 des Schnittblocks 2 eingesetzt werden können. Bei aufgesetzter Sägeblattführung 32 kann ein nicht dargestelltes Sägeblatt zur Resektion der Tibia 1 in dem zwischen der Sägeblattführung 32 und dem Schnittblock 2 gebildeten Zwischenraum 65 geführt werden (Fig. 9).

### Bezugszeichenliste

- 1: Tibia
- 2: Schnittblock
- 3: Verstellhülse
- 4: Natürliche Tibiaplattform
- 5: Querarm
- 6: erste Geradführung
- 7: erstes Schwenkelement
- 8: erste Achse
- 9: zweites Schwenkelement
- 10: zweite Achse
- 11: zweite Geradführung, Schlittenführung
- 12, 12a: Skala
- 13, 13a: Auflagefläche
- 15: Führungsbohrung
- 18, 18a: Stift
- 19: Verstellmutter
- 20, 20a: Stift
- 21: Führungsbohrung
- 22: intramedullärer Nagel
- 24: erstes Winkelstück
- 25: zweites Winkelstück
- 26: Schlitz
- 27: Flachstück
- 28: Basisplatte
- 29: Schlitten
- 30: Kipphebel
- 32: Sägeblattführung
- 33: Zahn
- 34: Wulst
- 35: Querträger
- 36: Achse
- 37: Rückstellfeder
- 38: Gewinde
- 39: Stützfläche
- 40: Nut
- 41: Klemmschraube
- 42: Anpressbacken
- 43: Klemmfeder
- 44: Platte
- 45: Einrastkugel
- 46: Druckknopf
- 47: Klinke
- 48: Rückstellfeder
- 49: Sicherungsstift
- 51: Langloch
- 52: Klinke
- 54: Schaft
- 55: Verzahnung
- 56: Kerbstift
- 57: Druckknopf
- 58: Rückstellfeder
- 59: Führungsbohrung
- 60: Zylinderstift
- 61: Verlängerung
- 62: Verzahnung
- 63: sphärische Vertiefung
- 64: Führungsbohrung
- 65: Zwischenraum

## Patentansprüche

1. Vorrichtung zum Setzen eines Schnittblocks (2) für eine Resektion der Tibia (1), welche sowohl extramedullär als auch intramedullär an der Tibia verankerbar ist und ein insbesondere rohrförmiges Verstellorgan (3) aufweist, mit welchem ein zur Verankerung an einer natürlichen Tibiaplattform (4) vorgesehener Querarm (5) verbunden ist, wobei der Schnittblock (2) an einer ersten Geradführung (6) relativ zum Querarm (5) in seiner Höhe verstellbar ist, wobei
für die erste Geradführung (6) eine Gelenkanordnung (8, 10) vorgesehen ist, durch die unterschiedliche Relativstellungen zwischen erster Geradführung (6), Querarm (5) und Verstellorgan (3) realisierbar sind,
die Gelenkanordnung wenigstens zwei Schwenkachsen (8, 10) umfasst,
die erste Schwenkachse (8) in anterior-/posterior-Richtung und die zweite Schwenkachse (10) in medio-lateraler Richtung orientiert ist,
der Querarm (5) einen Querträger (35) und ein um die erste Achse (8) relativ zum Querträger (35) schwenkbares erstes Schwenkelement (7) umfasst, das an der natürlichen Tibiaplattform (4) verankerbar ist,
relativ zum Querträger (5) ein zweites Schwenkelement (9) um die zweite Achse (10) verschwenkbar ist, mit welchem die erste Geradführung (6) verbunden ist, und
das erste Schwenkelement (7) zumindest zum Teil in Form eines Rahmens ausgeführt ist, welcher den Querträger (35) einschließt.

2. Vorrichtung nach Anspruch 1,
**dadurch gekennzeichnet ,**
**dass** die Schwenkachsen (8, 10) senkrecht zueinander orientiert sind.

3. Vorrichtung nach Anspruch 1 oder 2,
**dadurch gekennzeichnet ,**
**dass** die Schwenkachsen (8, 10) oberhalb der ersten Geradführung (6) verlaufen.

4. Vorrichtung nach einem der vorhergehenden Ansprüche,
**dadurch gekennzeichnet ,**
**dass** sich die Schwenkachsen (8, 10) schneiden.

5. Vorrichtung nach einem der vorhergehenden Ansprüche,
**dadurch gekennzeichnet ,**
**dass** der Querträger (35) und das Verstellorgan (3) miteinander verbunden sind.

6. Vorrichtung nach einem der vorhergehenden Ansprüche,
**dadurch gekennzeichnet ,**
**dass** zwischen der ersten Geradführung (6) zur Höhenverstellung des Schnittblocks (2) und dem zweiten Schwenkelement (9) eine zweite Geradführung (11) zur Verstellung des Schnittblocks (2) in anterior/posterior-Richtung angebracht ist.

7. Vorrichtung nach einem der vorhergehenden Ansprüche,
**dadurch gekennzeichnet ,**
**dass** der Winkel einer Schwenkbewegung um eine erste und/oder zweite Achse (8, 10) auf einer Skala (12, 12a) ablesbar ist.

8. Vorrichtung nach einem der vorhergehenden Ansprüche,
**dadurch gekennzeichnet ,**
**dass** der Winkel einer Schwenkbewegung um eine erste und/oder zweite Achse (8, 10) mit Formschluss oder Kraftschluss festsetzbar ist.

9. Vorrichtung nach einem der vorhergehenden Ansprüche,
**dadurch gekennzeichnet ,**
**dass** das erste Schwenkelement (7) zwei im gesetzten Zustand gegen distal vorstehende Stifte (20, 20a) zur Verankerung an der natürlichen Tibiaplattform (4) und eine Führungsbohrung (21) für den Fall einer Verankerung mit Ausrichtung an einem intramedullären Nagel (22) aufweist.

10. Vorrichtung nach einem der vorhergehenden Ansprüche,
**dadurch gekennzeichnet ,**
**dass** der Querträger (35) des Querarms (5) mit einem in Achsrichtung des Verstellorgans (3) vorstehenden ersten Winkelstück (24) versehen ist, das mit einem vom ersten Schwenkelement (7) vorstehenden zweiten Winkelstück (25) verriegelbar ist.

11. Vorrichtung nach einem der vorhergehenden Ansprüche,
**dadurch gekennzeichnet ,**
**dass** das zweite Schwenkelement (9) ein in einem Schlitz (26) des Querträgers (35) geführtes und schwenkbares Flachstück (27) umfasst, welches in eine Basisplatte (28) übergeht.

12. Vorrichtung nach Anspruch 11,
**dadurch gekennzeichnet ,**
**dass** an der Basisplatte (28) eine Schlittenführung (11) in anterior/posterior-Richtung für ein als Schlitten (29) ausgeführtes Trägerelement der ersten Geradführung (6) für den Schnittblock (2) vorhanden ist.

13. Vorrichtung nach einem der vorhergehenden Ansprüche, und Schnittblock,
**dadurch gekennzeichnet ,**
**dass** die beiden in medio-lateraler Richtung gelegenen Enden des Schnittblocks (2) voneinander verschieden ausgebildet sind.

14. Vorrichtung nach einem der Ansprüche 1 bis 12, und Schnittblock nach Anspruch 13,
**dadurch gekennzeichnet ,**
**dass** das eine Ende einen gegen posterior vorstehenden Wulst (34) aufweist.

15. Vorrichtung nach einem der Ansprüche 1 bis 12, und Schnittblock insbesondere nach Anspruch 13 oder 14,
**dadurch gekennzeichnet ,**
**dass** obere und untere Auflageflächen (13, 13a) des Schnittblocks (2) sowie im Schnittblock (2) ausgebildete Führungsbohrungen (15) für Verankerungsstifte bezüglich einer transversalen Mittelebene des Schnittblocks (2) symmetrisch ausgebildet sind.

16. Vorrichtung nach einem der Ansprüche 1 bis 12, und Schnittblock insbesondere nach einem der Ansprüche 13 bis 15,
**dadurch gekennzeichnet ,**
**dass** der Schnittblock (2) Führungsbohrungen (64) für eine Sägeblattführung (32) aufweist.

## Claims

1. An apparatus for the setting of a cutting block (2) for a resection of the tibia (1) which can be anchored to the tibia both in an extrame-dullary and an intramedullary fashion and which has an adjustment member (3), in particular a tubular adjustment member, to which a transverse arm (5) provided for anchorage to a natural tibia platform (4) is connected, with the cutting block (2) being adjustable in its height relative to the transverse arm (5) at a first straight guide (6), wherein
a joint arrangement (8, 10) is provided for the first straight guide (6) by which different relative positions can be realized between the first straight guide (6), the transverse arm (5) and the adjustment member (3);
the joint arrangement includes at least two pivot axes (8, 10); the first pivot axis (8) is oriented in the anterior/posterior direction and the second pivot axis (10) is oriented in the medio-lateral direction;
the transverse arm (5) includes a transverse carrier (35) and a first pivot element (7) which is pivotable around the first axis (8) relative to the transverse carrier (35) and can be anchored to the natural tibia platform (4);
a second pivot element (9) is pivotable around the second axis (10) relative to the transverse carrier (5) and the first straight guide (6) is connected to it; and
the first pivot element (7) is made at least partly in the form of a frame which includes the transverse carrier (35).

2. A system in accordance with claim 1, **characterized in that** the pivot axes (8, 10) are oriented perpendicular to one another.

3. An apparatus in accordance with claim 1 or claim 2, **characterized in that** the pivot axes (8, 10) extend above the first straight guide (6).

4. An apparatus in accordance with any one of the preceding claims, **characterized in that** the pivot axes (8, 10) intersect.

5. An apparatus in accordance with any one of the preceding claims, **characterized in that** the transverse carrier (35) and the adjustment member (3) are connected to one another.

6. An apparatus in accordance with any one of the preceding claims, **characterized in that** a second straight guide (11) for the adjustment of the cutting block (2) in the anterior/posterior direction is attached between the first straight guide (6) for the vertical adjustment of the cutting block (2) and the second pivot element (9).

7. An apparatus in accordance with any one of the preceding claims, **characterized in that** the angle of a pivotal movement about a first and/or second axis (8, 10) can be read off at a scale (12, 12a).

8. An apparatus in accordance with any one of the preceding claims, **characterized in that** the angle of a pivotal movement about a first and/or second axis (8, 10) can be fixed by shape matching or by force transmission.

9. An apparatus in accordance with any one of the preceding claims, **characterized in that** the first pivot element (7) has two pins (20, 20a), projecting toward distal in the set state, for the anchorage to the natural tibia platform (4) and a guide bore (21) for the event of an anchorage with alignment at an intramedullary nail (22).

10. An apparatus in accordance with any one of the preceding claims, **characterized in that** the transverse carrier (35) of the transverse arm (5) is provided with a first bracket piece (24) projecting in the axial direction of the adjustment member (3) and is latchable to a second bracket piece (25) projecting from the first pivot element (7).

11. An apparatus in accordance with any one of the preceding claims, **characterized in that** the second pivot element (9) includes a flat piece (27) which is guided and pivotable in a slot (26) of the transverse carrier (35) and which merges into a base plate (28).

12. An apparatus in accordance with claim 11, **characterized in that** a carriage guide (11) is present at the base plate (28) in the anterior/posterior direction for a carrier element of the first straight guide (6) for the cutting block (2), the carrier element being designed as a carriage (29).

13. An apparatus in accordance with any one of the preceding claims, **characterized in that** the two ends of the cutting block (2) disposed in the medio-lateral direction are made different from one another.

14. An apparatus in accordance with any one of the claims 1 to 12 and a cutting block in accordance with claim 13, **characterized in that** the one end has a bead (34) projecting toward posterior.

15. An apparatus in accordance with any one of the claims 1 to 12 and a cutting block in particular in accordance with claim 13 or claim 14, **characterized in that** upper and lower support surfaces (13, 13a) of the cutting block (2) and guide bores (15) for anchorage pins formed in the cutting block (2) are made symmetrical with respect to a transverse central plane of the cutting block (2).

16. An apparatus in accordance with any one of claims 1 to 12 and a cutting block in particular in accordance with any one of the claims 13 to 15, **characterized in that** the cutting block (2) has guide bores (64) for a saw blade guide (32).

## Revendications

1. Dispositif pour la pose d'un guide de coupe (2) pour une résection du tibia (1), qu'il est possible d'ancrer sur le tibia aussi bien de manière extramédullaire que de manière intramédullaire, et qui comporte un organe de réglage (3), en particulier de forme tubulaire, auquel est relié un bras transversal (5) prévu pour l'ancrage sur une plate-forme tibiale naturelle (4), le guide de coupe (2) étant réglable quant à sa hauteur par rapport au bras transversal (5) sur un premier guidage rectiligne (6), dans lequel
pour le premier guidage rectiligne (6), il est prévu un premier agencement d'articulation (8, 10) au moyen duquel différentes positions relatives entre le premier guidage rectiligne (6), le bras transversal (5) et l'organe de réglage (3) sont réalisables,
l'agencement d'articulation comprend au moins deux axes de pivotement (8, 10), le premier axe de pivotement (8) étant orienté en direction antérieure/postérieure et le second axe de pivotement (10) étant orienté en direction médiale-latérale,
le bras transversal (5) comprend un support transversal (35) et un premier élément pivotant (7), capable de pivoter autour du premier axe (8) par rapport au support transversal (35), qui est susceptible d'être ancré sur la plate-forme tibiale naturelle (4),
un second élément pivotant (9) est capable de pivoter par rapport au support transversal (5) autour du deuxième axe (10), le premier guidage rectiligne (6) étant relié audit second élément pivotant, et
le premier élément pivotant (7) est réalisé au moins en partie sous la forme d'un cadre qui enferme le support transversal (35).

2. Dispositif selon la revendication 1,
**caractérisé en ce que** les axes de pivotement (8,10) sont orientés perpendiculairement l'un à l'autre.

3. Dispositif selon la revendication 1 ou 2,
**caractérisé en ce que** les axes de pivotement (8, 10) s'étendent au-dessus du premier guidage rectiligne (6).

4. Dispositif selon l'une des revendications précédentes,
**caractérisé en ce que** les axes de pivotement (8, 10) se recoupent.

5. Dispositif selon l'une des revendications précédentes,
**caractérisé en ce que** le support transversal (35) et l'organe de réglage (3) sont reliés l'un à l'autre.

6. Dispositif selon l'une des revendications précédentes,
**caractérisé en ce qu'**un second guidage rectiligne (11), destiné au réglage du guide de coupe (2) dans la direction antérieure/postérieure, est monté entre le premier guidage rectiligne (6) pour le réglage en hauteur du guide de coupe (2) et le second élément pivotant (9).

7. Dispositif selon l'une des revendications précédentes,
**caractérisé en ce que** l'angle d'un mouvement de pivotement autour d'un premier et/ou d'un second axe (8, 10) est lisible sur une échelle (12, 12a).

8. Dispositif selon l'une des revendications précédentes,
**caractérisé en ce que** l'angle d'un mouvement de pivotement autour d'un premier et/ou d'un second axe (8, 10) est susceptible d'être fixé par coopération de formes ou par coopération de forces.

9. Dispositif selon l'une des revendications précédentes,
**caractérisé en ce que** le premier élément pivotant (7) comprend deux tiges (20, 20a) saillantes en direction distale à l'état posé, pour l'ancrage sur la plate-forme tibiale naturelle (4), et un perçage de guidage (21) pour le cas d'un ancrage avec alignement sur une broche intramédullaire (22).

10. Dispositif selon l'une des revendications précédentes,
**caractérisé en ce que** le support transversal (35) du bras transversal (5) est pourvu d'une première équerre (24) qui dépasse dans la direction axiale de l'organe de réglage (3) et qui peut être verrouillée avec une seconde équerre (25) qui dépasse depuis le premier élément pivotant (7).

11. Dispositif selon l'une des revendications précédentes,
**caractérisé en ce que** le second élément pivotant (9) comprend une pièce plate (27) guidée dans une fente (26) du support transversal (35) et capable de pivoter, qui se transforme dans une plaque de base (28).

12. Dispositif selon la revendication 11,
**caractérisé en ce qu'**il est prévu un guidage de chariot (11) en direction antérieure/postérieure sur la plaque de base (28) pour un élément de support, réalisé sous forme de chariot (29), du premier guidage rectiligne (6) pour le guide de coupe (2).

13. Dispositif selon l'une des revendications précédentes, avec un guide de coupe,
**caractérisé en ce que** les deux extrémités du guide de coupe (2) situées en direction médiale-latérale sont réalisées de manière différente l'une par rapport à l'autre.

14. Dispositif selon l'une des revendications 1 à 12, avec un guide de coupe, selon la revendication 13,
**caractérisé en ce que** l'une des extrémités comporte un bourrelet (34) en dépassement en direction postérieure.

15. Dispositif selon l'une des revendications 1 à 12, avec un guide de coupe en particulier selon la revendication 13 ou 14,
**caractérisé en ce que** des surfaces d'appui supérieure et inférieure (13, 13a) du guide de coupe (2), ainsi que des perçages de guidage (15) ménagés dans le guide de coupe (2) pour des tiges d'ancrage, sont réalisés de façon symétrique par rapport à un plan médian transversal du guide de coupe (2).

16. Dispositif selon l'une des revendications 1 à 12, avec un guide de coupe en particulier selon l'une des revendications 13 à 15,
**caractérisé en ce que** le guide de coupe (2) comporte des perçages de guidage (64) pour le guidage d'une lame de scie (32).
